# EUROPÄISCHE PATENTSCHRIFT

(11) **EP 4 203 305 B1**
(45) Veröffentlichungstag und Bekanntmachung des Hinweises auf die Patenterteilung: **13.03.2024**
(21) Anmeldenummer: 21217016.1
(22) Anmeldetag: 22.12.2021
(51) Int. Cl.: H03F 1/02, A61B 18/12

(54) **GENERATOR ZUR VERSORGUNG MEDIZINISCHER INSTRUMENTE**
GENERATOR FOR SUPPLYING MEDICAL INSTRUMENTS
GÉNÉRATEUR DESTINÉ À L'ALIMENTATION DES INSTRUMENTS MÉDICAUX

(43) Veröffentlichungstag der Anmeldung: 28.06.2023
(73) Patentinhaber: Erbe Elektromedizin GmbH, 72072 Tübingen (DE)
(72) Erfinder: DORNHOF, Konstantin, 72160 Horb am Neckar (DE)
(74) Vertreter: Rüger Abel Patentanwälte PartGmbB

(56) Entgegenhaltungen:
- EP-A1- 0 949 886
- EP-A2- 2 753 259
- US-A1- 2003 163 124

## Beschreibung

Die Erfindung, welche im Anspruch 1 definiert ist, betrifft einen Generator zur Versorgung elektrochirurgischer Instrumente, insbesondere einen Generator mit verbesserter Kontrolle über die vom Generator gelieferte Ausgangsspannung.

Viele elektrochirurgische Instrumente, wie beispielsweise Elektroskalpelle, elektrochirurgische Zangen oder Koagulationsinstrumente, werden typischerweise mit einem hochfrequenten Wechselstrom gespeist. Zur Vermeidung neuromuskulärer Stimulationen liegt die Frequenz dieser Wechselspannung typischerweise oberhalb von 100 kHz. Die Leistung solcher Generatoren liegt typischerweise deutlich über 1 W und kann mehrere 100 W erreichen. Ein Generator nach der Präambel von Anspruch 1 ist aus EP 0 949 886 A1 bekannt.

Ein Generator zur Erzeugung solcher Spannungen kann der DE 10 2008 039 884 A1 entnommen werden. Dieser Generator enthält mindestens einen Schwingkreis, der über eine aktive Transistorschaltung zur Schwingung erregt wird und aus dem die Energie zur Speisung des angeschlossenen Instruments transformatorisch ausgekoppelt wird.

Ein Generator zur gleichzeitigen Speisung mehrerer Instrumente ist beispielsweise der DE 60 2004 009 293 T2 zu entnehmen. Dieser Generator beinhaltet mehrere Schwingkreise, die an eine Vollbrückenschaltung angeschlossen sind.

Die DE 29 10 196 A offenbart einen Gegentaktoszillator mit zwei im Gegentakt arbeitenden Transistoren, zwischen deren Kollektoren ein Parallelschwingkreis angeordnet ist. Über eine mit der Schwingkreisspule transformatorisch koppelnde Wicklung werden Spannung und Strom für ein chirurgisches Instrument ausgekoppelt.

Es hat sich herausgestellt, dass die Kurvenform des von dem Generator an das Instrument gelieferten Stroms erhebliche Auswirkung auf die zu erzielende physiologische Wirkung hat. Deswegen besteht der Wusch nach einer verbesserten Beeinflussungsmöglichkeit der Kurvenform der Ausgangsspannung und des Ausgangsstroms eines Generators zur Speisung eines Instruments.

Davon leitet sich die der Erfindung zugrundeliegende Aufgabe ab, einen Generator mit erweiterten Möglichkeiten zur Beeinflussung der Strom- und Spannungsform zu schaffen.

Diese Aufgabe wird mit einem Generator nach Anspruch 1 gelöst:
Der erfindungsgemäße Generator beruht auf einem Konzept mit wenigstens zwei Verstärkern, zwischen deren Ausgängen ein Induktor eines Resonanzkreises angeordnet ist. Dem Induktor ist mindestens ein erster Kondensator zugeordnet, mit dem er einen Schwingkreis bildet, sowie ein zweiter Kondensator zur weiteren Beeinflussung der Resonanzfrequenz des Schwingkreises. Vorzugsweise ist dabei wenigstens der zweite Kondensator fest oder über einen Schalter, vorzugsweise einen elektronischen Schalter, mit Masse verbunden. Ist der Verstärker durch ein elektronisches Verstärkerelement, wie beispielsweise einen Bipolartransistor oder einen Feldeffekttransistor gebildet, ist eine Elektrode dieses Kondensators mit der Ausgangselektrode des entsprechenden Transistor (Collector oder Drain) und die andere Elektrode des Kondensators mit Masse verbunden. Insoweit ist der betreffende frequenzbeeinflussende Kondensator dem Verstärkerelement ausgangsseitig parallel geschaltet. Insbesondere wenn das Verstärkerelement im Schaltbetrieb arbeitet, bewirkt es somit den zeitweiligen Kurzschluss des betreffenden Kondensators, der somit zeitweilig unwirksam ist. Der Kondensator beeinflusst nur diejenige Halbewelle der an dem Schwingkreis angefachten Schwingung, bei der das Verstärkerelement, dem er parallel geschaltet ist, nicht leitend (hochohmig) ist.

Der Generator ist als Gegentaktoszillator aufgebaut. Die beiden Verstärker sind an eine Steuerschaltung angeschlossen, die darauf eingerichtet ist, die Verstärker gegensinnig zu steuern. Vorzugsweise ist die Steuerschaltung dabei darauf eingerichtet, die Verstärker im Schaltbetrieb zu steuern. Das heißt, die entsprechenden Verstärkerelemente (Transistoren) sind entweder stromdurchlässig, d.h. niederohmig leitend oder gesperrt.

Das erfindungsgemäße Schaltungskonzept gestattet die gezielte Einflussnahme auf Amplitude und Dauer einer einzelnen Halbwelle eines solchen Generators. Damit können beispielsweise gezielt die positiven (oder auch die negativen Halbwellen) der Generatorschwingung in ihrer Amplitude vergrößert oder verkleinert sowie zeitlich verlängert oder verkürzt werden. Werden mehrere solcher zusätzlichen Kondensatoren mit entsprechenden Schaltern an den Schwingkreis und den zugeordneten Verstärkerausgang angeschlossen, kann die entstehende Generatorschwingung vielfältig beeinflusst werden, sodass auch anderweitig sonst nicht erzielbare Ausgangskurvenformen der vom Generator gelieferten Spannung oder des vom Generator gelieferten Stroms erzielbar sind. Beispielsweise kann die an das Instrument gelieferte Schwingung gezielt asymmetrisch erzeugt werden, um beispielsweise die Funkenbildung an einer Instrumentenelektrode zu fördern oder zu hemmen.

Die beiden Verstärker können, wie erwähnt, durch elektronische Schalter gebildet sein, die durch eine Steuerschaltung vorzugsweise im Gegentakt angesteuert sind. Der Induktor kann mit einer Mittenanzapfung versehen sein, über die dem Generator und somit den Verstärkern Betriebsstrom zugeführt wird. Der Generator ist nach diesem Konzept ein symmetrischer Gegentaktoszillator, der durch Zu- und Abschaltung von Kondensatoren asymmetriert werden kann. Auch eine asymmetrische Speisung, insbesondere hochfrequenzmäßig hochohmige asymmetrische Speisung ist möglich.

Einzelheiten erfindungsgemäßer Schaltungsvarianten sind Gegenstand von Unteransprüchen sowie der Zeichnung und der zugehörigen Beschreibung deren Figuren. In der Zeichnung zeigen:
Figur 1 einen Generator und einen angeschlossenes Instrument, in schematisierter Übersichtsdarstellung,
Figur 2 einen Generator zur Speisung des Instruments, in schematisierter Schaltungsdarstellung,
Figur 3 die Ausgangsspannung des Generators nach Figur 2 als schematisches Diagramm,
Figur 4 bis 7 abgewandelte Ausführungsformen des Generators, jeweils im Prinzipschaltbild.

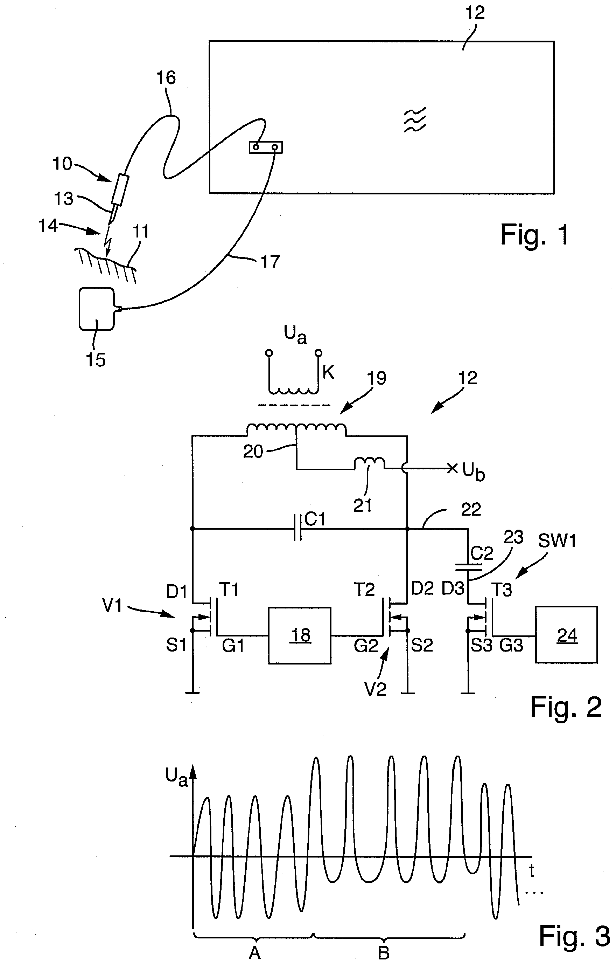

In Figur 1 sind ein Instrument 10 zur Behandlung von biologischem Gewebe 11 sowie ein Generator 12 zur Speisung des Instruments 10 veranschaulicht. Im Beispiel ist ein monopolares Instrument 10 mit einer einzigen Elektrode 13 dargestellt, die mittels eines Funkens 14 auf das biologische Gewebe 11 einwirkt. Zur Rückführung des von dem Instrument 10 bzw. der Elektrode 13 ausgehenden Stroms zu dem Generator 12 ist eine Neutralelektrode 15 vorgesehen, die an dem menschlichen oder tierischen Patienten anzubringen ist, zu dem das Gewebe 11 gehört. Leitungen 16, 17 verbinden das Instrument 10 und die vorzugsweise großflächige Neutralelektrode 15 mit dem Generator 12. Diese Anordnung ist beispielhaft zu verstehen. Der Generator 12 ist ebenso zur Speisung bipolarer Instrumente geeignet, wie beispielsweise zur Speisung von Koagulationswerkzeugen mit zwei oder mehreren Elektroden. Dies insbesondere, weil der Generator 12 in besonderer Weise dazu geeignet ist, eine Ausgangsspannung Ua mit einer Kurvenform zu liefern, die sich an verschiedene physiologische Gegebenheiten und chirurgische Anforderungen und Aufgaben anpassen lässt.

Figur 2 veranschaulicht die Prinzipschaltung des Generators 12. Er umfasst einen ersten Verstärker V1 und einen zweiten Verstärker V2, die durch Transistoren T1, T2 gebildet sein können. Die Transistoren T1, T2 sind vorzugsweise Feldeffekttransistoren, wie beispielsweise n-MOS-Transistoren vom Anreicherungstyp oder vom Verarmungstyp. Andere Transistoren, insbesondere Bipolartransistoren, IG-BTs (Bipolartransistoren mit isoliertem Gate) und dergleichen können ebenfalls Anwendung finden. Sind die Transistoren T1 und T2 Feldeffekttransistoren, weisen sie jeweils ein Gate G1, G2, eine Sourceelektrode S1, S2 sowie eine Drainelektrode D1, D2 auf. Die beiden Transistoren T1, T2 arbeiten in Sourceschaltung, sodass die Gates G1, G2 die Eingangselektroden der Verstärker V1, V2 bilden. Die Drainelektroden D1, D2 bilden dann die Ausgangselektroden der Verstärker V1, V2.

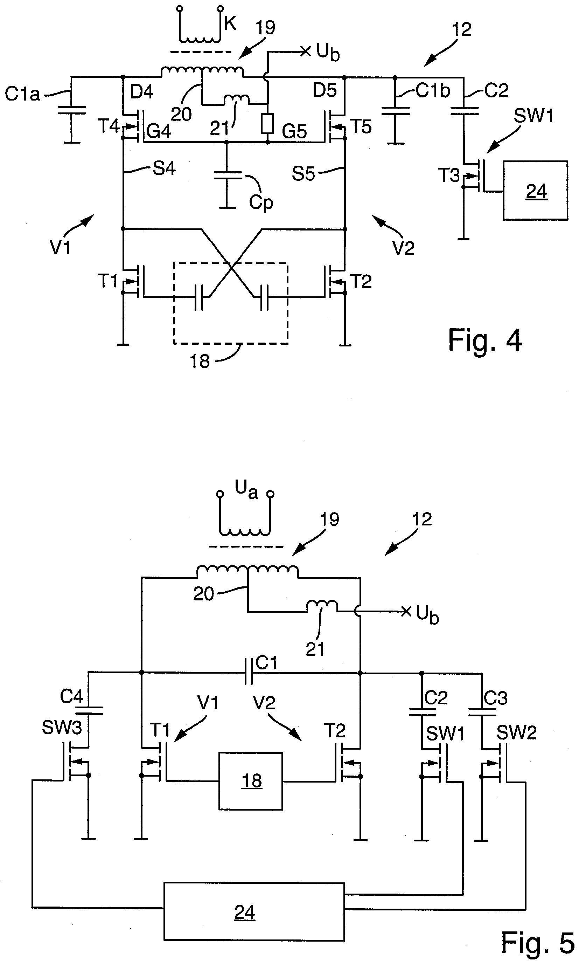

Die Gates G1, G2 sind mit einer Steuerschaltung 18 verbunden, die dazu eingerichtet ist, die Transistoren T1, T2 im Takt mit der Frequenz der zu erzeugenden Wechselspannung gegensinnig zu öffnen und zu schließen, d.h. leitend und nicht leitend zu machen. Die Transistoren T1, T2 werden somit im Gegentakt gesteuert und arbeiten im Schaltbetrieb. Die Steuerschaltung 18 kann bei allen Ausführungsformen des Generators 12, wie es in Figur 4 beispielhaft dargestellt ist, durch zwei Koppelkondensatoren gebildet sein, über die jeweils ein Gate eines der Transistoren mit dem Drain des jeweils anderen Transistors verbunden ist. Diese Art der Kopplung bewirkt, dass immer einer der Transistoren T1, T2 leitet, während der andere der beiden Transistoren T1, T2 sperrt. Die Sperr- und Leitphasen wechseln mit der von dem Schwingkreis vorgegebenen Frequenz. Die beiden Koppelkondensatoren sind vorzugsweise gleich groß.

Die Sourceelektroden S1, S2 der beiden Verstärker V1, V2 (Transistoren T1, T2) sind direkt oder zumindest wechselstrommäßig mit Masse verbunden. Die Ausgangselektroden der beiden Verstärker V1, V2, d.h. die Drains D1, D2, der Transistoren T1, T2 sind untereinander durch einen Induktor 19 verbunden, der zumindest vorzugsweise eine Mittenanzapfung 20 aufweist, über die dem Induktor 19 und somit auch den Verstärkern V1, V2 Betriebsspannung Ub und damit auch entsprechend Betriebsstrom zugeführt wird. In der Zuführungsleitung zu der Mittenanzapfung 20 können eine Drossel 21 sowie gegebenenfalls weitere Siebmittel vorgesehen sein. Die Drossel 21 sperrt einen Abfluss hochfrequenten Stroms zu der Betriebsspannungsquelle hin und vermeidet so eine unerwünschte Dämpfung der Schwingung des Induktors.

Der Generator 12 enthält außerdem einen ersten Kondensator C1, der dem Induktor 19 parallel geschaltet ist, um mit diesem einen Parallelschwingkreis zu bilden. Auf diese Weise verbindet der erste Kondensator C1 die Ausgangselektroden der Verstärker V1 und V2, d.h. die Drains D1 und D2 miteinander.

Der insoweit beschriebene Generator 12 ist in der bis hier her beschriebenen Konfiguration prinzipiell schwing- und betriebsfähig. Jedoch weist er zusätzliche Mittel zur Beeinflussung seines Betriebs auf. Dazu gehört ein zweiter Kondensator C2, der mit einem Ende 22 an den aus dem Induktor 16 und dem Kondensator C1 gebildeten Parallelschwingkreis und mit einem anderen Ende 23 fest oder, wie in Figur 2 dargestellt, über einen ersten Schalter SW1 an Masse angeschlossen ist.

Der Schalter SW1 kann ein elektronischer Schalter beispielsweise in Gestalt eines Transistors T3 sein, der beispielsweise als Feldeffekttransistor ausgebildet sein kann. Er kann vom gleichen Typ wie die Transistoren T1, T2 oder auch von einem anderen Typ sein. Seine Gateelektrode G3 ist an eine Steuerschaltung 24 angeschlossen, die den Schalter SW1 gezielt für mindestens eine Schwingungsperiode oder auch für eine längere Zeitspanne öffnet oder schließt.

Der insoweit beschriebene Generator 12 arbeitet wie folgt:
Die Leitungen 16, 17 sind mit der vom Generator 12 erzeugten Spannung Ua verbunden, indem die Leitungen 16, 17 zu einer Koppelspule K geführt sind, die induktiv mit dem Induktor 19 verbunden ist. Der aus dem Induktor 19 und dem Kondensator C1 gebildete Parallelschwingkreis wird zum Schwingen erregt, indem die beiden Verstärker V1, V2 im Gegentakt betreiben werden. Dazu werden die Transistoren T1, T2 abwechselnd leitend und nicht leitend. Dies wird von der Steuerschaltung 18 gesteuert. Die Frequenz der Ansteuerung der Transistoren T1, T2 stimmt vorzugsweise mit der Resonanzfrequenz des Parallelschwingkreises (Induktor 19 und Kondensator C1) überein.

Es wird zunächst angenommen, dass der Schalter SW1 nicht leitend, d.h. der Transistor T3 gesperrt ist. Damit ist der wirksame Teil der Schaltung des Generators 12 symmetrisch aufgebaut, der Kondensator C2 ist unwirksam. Es wird eine symmetrische Schwingung erzeugt, wie in Figur 3 im Diagramm in der zeitlichen Periode A dargestellt ist.

Die erzeugte symmetrische Ausgangsspannung Ua liegt zunächst als unmodulierte Ausgangsspannung an. Bedarfsweise kann eine Amplitudenmodulation vorgenommen werden, beispielsweise durch Modulation der Betriebsspannung Ub. Für die nachfolgenden Betrachtungen wird jedoch angenommen, dass die Betriebsspannung Ub konstant ist.

Die Ausgangsspannung Ua in dem Zeitabschnitt A hat eine bestimmte physiologische Wirkung, wenn das damit gespeiste Instrument 10 auf das Gewebe 11 einwirkt, beispielsweise eine Koagulationswirkung. Es wird nun angenommen, dass eine andere physiologische Wirkung gewünscht und der Generator 12 deswegen eine Ausgangsspannung Ua mit einer anderen Kurvenform erzeugen soll. Insbesondere soll diese beispielsweise unterschiedliche Schwingungsverläufe in der positiven und der negativen Halbwelle haben. Beispielsweise können sich die Halbwellen in zeitlicher Länge und in der Größe (Amplitude) unterscheiden. Dazu schließt die Steuerschaltung 24 den Schalter SW1, d.h. sie macht den Transistor T3 zwischen seiner Sourceelektrode D3 und seiner Drainelektrode D3 leitend. So wird das Ende 23 des Kondensators C2 mit Masse verbunden. Diese elektrische Verbindung wird über die gesamte in Figur 3 dargestellte Zeitspanne B aufrechterhalten.

In der Halbwelle, in der der Transistor T2 leitend ist, ist der zweite Kondensator C2 unwirksam. In derjenigen Halbwelle aber, in der T1 leitend und der Transistor T2 nicht leitend ist, ist der Kondensator C2 wirksam und verbindet den Verbindungspunkt zwischen dem ersten Kondensator C1 und dem Induktor 19 mit Masse. Dadurch kann eine in Amplitude und Frequenz veränderte, beispielsweise eine kleinere, dafür aber längere Halbwelle entstehen. Diese Schwingung des Generators 12 kann dann beispielsweise die in Figur 3 in den Zeitabschnitt B gezeigte Form annehmen. Ist die positive Halbwelle über die Leitung 16 mit der Elektrode 13 verbunden, wird dadurch die Funkenbildung zwischen der Elektrode 13 und dem biologischen Gewebe 11 gefördert und somit ein anderer physiologischer Effekt erzielt, als in dem Zeitabschnitt A.

Figur 4 veranschaulicht eine abgewandelte Ausführungsform des Generators 12, die sich von der vorstehend beschriebenen Ausführungsform lediglich darin unterscheidet, dass die Verstärker V1, V2 durch Kaskodeschaltungen aus Transistoren T1, T2, T4, T5 gebildet sind. Die Transistoren T1, T2 entsprechen den Transistoren T1, T2 nach Figur 2. Die Transistoren T4, T5 sind in Gateschaltung geschaltet und bilden somit Endverstärker mit ihren Sourceelektroden S4, S5 als Eingangselektroden, wohingegen ihre Gateelektroden G4, G5 auf einem geeigneten festen Potential liegen, das über einen Kondensator Cp wechselstrommäßig mit Masse verbunden ist.

Der Kondensator C1 ist hier in zwei einzelne Kondensatoren C1a, C1b aufgeteilt, die jeweils mit einem Ende auf Masse liegen. Das jeweilige andere Ende jedes Kondensators C1a, C1b ist mit den beiden Enden des Induktors 19 verbunden. Wiederum bilden die Kondensatoren C1a, C1b mit dem Induktor 19 einen Parallelschwingkreis. Der Schalter SW1 dient, wie schon bei der in Verbindung mit Figur 2 beschriebenen Schaltung, zur Veränderung des Betriebs des Generators 12. Insbesondere kann er eine Asymmetrierung der Schwingung des Generators 12 bewirken, damit dieser bedarfsweise zwischen der Schwingungsform des Zeitabschnitts A nach Figur 3 und des Zeitabschnitts B nach Figur 3 umgeschaltet werden kann.

Unabhängig davon, ob der Generator 12 ein einfacher Gegentaktgenerator nach dem Konzept der Figur 2 oder ein Gegentaktgenerator mit Verstärkern V1, V2 in Kaskodeschaltung gemäß Figur 4 ist, kann er neben dem Schalter SW1 weitere Schalter SW2, SW3 mit Kondensatoren C3, C4 enthalten, um die Schwingungsform der Ausgangsspannung Ua gezielt zu beeinflussen. Ein solches Beispiel zeigt Figur 5. Beiden Transistoren T1, T2 und somit beiden Verstärkern V1, V2 sind ausgangsseitig Schaltzweige parallel geschaltet, in denen Kondensatoren C2, C3, C4 jeweils in Reihe mit einem Schalter SW1, SW2, SW3 gegen Masse angeordnet sind. Die Steuerschaltung 24 steuert die Schalter SW1, SW2, SW3 um damit einen oder mehrere der Kondensatoren C2, C3, C4 mit Masse zu verbinden und diesen dadurch wirksam zu machen.

Ebenso kann die Steuerschaltung 24 einen oder mehrere der Schalter SW1, SW2, SW3 öffnen (nicht leitend machen), um dadurch einen oder mehrere der Kondensatoren C2, C3, C4 unwirksam zu machen. Sind alle Kondensatoren C2, C3, C4 unwirksam, schwingt der Generator 12 nach dem Muster des Zeitabschnitts A in Figur 3. Werden ein oder mehrere der Kondensatoren C2, C3, C4 aktiviert, indem der jeweils zugeordnete Schalter SW1, SW2 oder SW3 geschlossen (leitend gemacht) wird, wird die Form der Ausgangsspannung Ua charakteristisch deformiert, beispielsweise indem die positive oder negative Halbwelle in ihrer Amplitude vergrößert oder verringert und zeitlich gestaucht oder gedehnt wird.

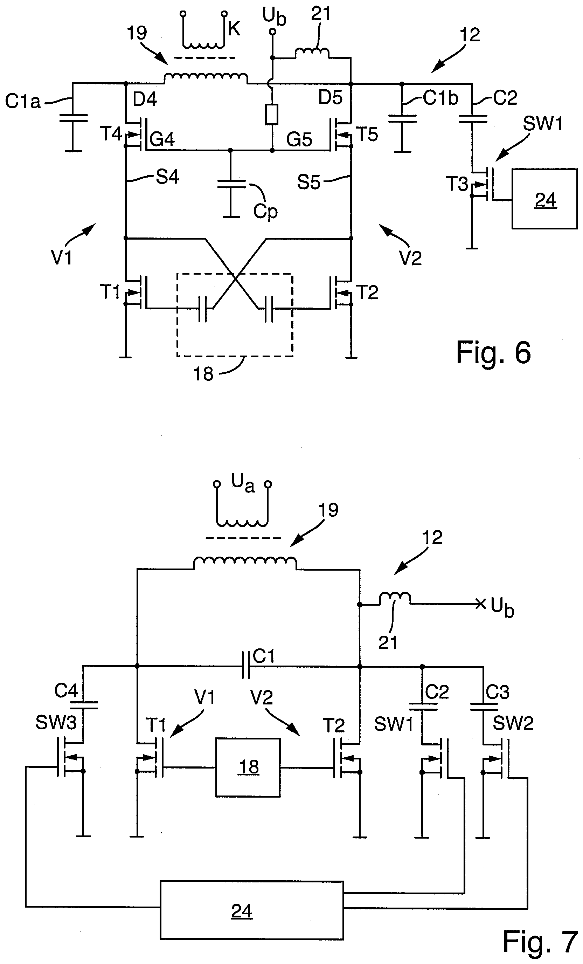

Bei allen beschriebenen Ausführungsformen können ein einziger Schalter SW1 mit dem Kondensator C2 (Figur 2, 6), zwei Schalter SW1, SW2 mit zwei Kondensatoren C2, C3, drei Schalter SW1, SW2 SW3 mit drei Kondensatoren C2, C3, C4 (Figur 5, 7) oder auch mehrere solcher Zweige vorgesehen sein.

Bei den vorstehend beschriebenen Schaltungskonzepten nach Figur 2 bis 5 weist der Induktor 19 jeweils die Mittenanzapfung 20 auf, an der Betriebsstrom über eine HFmäßig hochohmige Drossel 21 eingespeist wird. Damit ist eine symmetrische Stromeinspeisung gegeben. Bei allen vorgenannten Schaltungen ist es jedoch auch möglich, den Betriebsstrom über die Drossel 21 asymmetrisch an einem Ende des Induktors 19 einzuspeisen, wie insbesondere aus den Figuren 6 und 7 hervorgeht. Vorzugsweise ist die Induktivität der Drossel 21 dabei höher als die des Induktors 19. Wegen der Struktur und Funktionsbeschreibung dieser Schaltungen nach Figur 6 und 7 wird unter Zugrundelegung der bereits eingeführten Bezugszeichen auf die Beschreibung der Schaltungen nach Figur 4 und 5 verwiesen. Ergänzend gilt, dass durch die nach Figur 6 und 7 asymmetrische Einspeisung des Betriebsstroms in den Generator 12 eine Anfangs-Asymmetrie gegeben ist, die auch dann zu einer nicht vollständig symmetrischen Schwingung führen kann, wenn die zusätzlichen Kondensatoren C2, C3, C4 inaktiv, d.h. die zugehörigen Schalter SW1, SW2, SW3 gesperrt sind. Die vorhandene Anfangs-Asymmetrie der Schwingung des Generators 12 kann dann durch gezieltes Öffnen und Schließen eines oder mehrerer der Schalter SW1, SW2, SW3 verstärkt oder abgeschwächt werden.

Ein zur Versorgung eines medizinischen Instruments 10 vorgesehener Gegentaktgenerator 12 weist parallel zu mindestens einem seiner beiden Transistoren T1, T2 mindestens einen vorzugsweise schaltbar ausgebildeten kapazitiven Zweig gegen Masse auf. Ein solcher kapazitiver schaltbarer Zweig kann durch eine Reihenschaltung aus einem Kondensator C2 und einem Schalter SW1 bestehen. Damit kann eine der beiden Halbwellen der Ausgangsspannung des Generators 12 gezielt beeinflusst und die andere der beiden Halbwellen weitgehend unbeeinflusst gelassen werden. Sind beiden Transistoren T1, T2 schaltbare Zweige mit Kondensatoren C2, C4 parallel geschaltet, können beide Halbwellen der Ausgangsspannung Ua des Generators 12 unabhängig voneinander beeinflusst werden.

Das erfindungsgemäße Konzept gestattet somit die gezielte Einflussnahme auf Halbschwingungen eines sonst symmetrischen Gegentaktgenerators 12, womit das Einsatzspektrum bei der Versorgung medizinischer Instrumente 11 mit Behandlungsstrom vergrößert wird.

### Bezugszeichen:

- 10: Instrument
- 11: biologisches Gewebe
- 12: Generator
- 13: Elektrode
- 14: Funken
- 15: Neutralelektrode
- 16: Leitung des Instruments 10
- 17: Leitung der Neutralelektrode 15
- Ua: von dem Generator 12 an das Instrument 10 abgegebene Spannung
- V1, V2: Verstärker
- T1 - T5: Transistoren
- G1 - G5: Gateelektroden
- S1 - S5: Sourceelektroden
- D1 - D5: Drainelektroden
- 18: Steuerschaltung
- 19: Induktor
- 20: Mittenanzapfung
- C1: erster Kondensator
- C2: zweiter Kondensator
- Cp: Pufferkondensator
- 21: Drossel
- 22: erstes Ende des Kondensators C2
- 23: zweites Ende des Kondensators C2
- SW1: erster Schalter
- 24: Steuerschaltung des Schalters SW1 (SW2, SW3)
- K: Koppelspule
- A, B: Zeitabschnitte in Figur 3
- C4, C5: weitere Kondensatoren

## Patentansprüche

1. Generator (12) zur Speisung eines chirurgischen Instruments (10) mit elektrischem Strom,
mit einem ersten Verstärker (V1), der eine erste Eingangselektrode (G1) und eine erste Ausgangselektrode (D1) aufweist,
mit einem zweiten Verstärker (V2), der eine zweite Eingangselektrode (G2) und eine zweite Ausgangselektrode (D2) aufweist,
mit einem Induktor (19), der zwischen der ersten und der zweiten Ausgangselektrode (D1, D2) angeordnet ist,
mit einem ersten Kondensator (C1), der mit dem Induktor (19) zu einem Schwingkreis verbunden ist, **gekennzeichnet durch** einen zweiten Kondensator (C2), der ein mit dem Induktor (19) verbundenes Ende (22) und ein mit einem Schalter (SW1) verbundenes Ende (23) aufweist.

2. Generator nach Anspruch 1, **dadurch gekennzeichnet, dass** der erste Verstärker (V1) ein erster Feldeffekttransistor (T1) ist, dessen Drainelektrode die erste Ausgangselektrode (D1) ist, und dass der zweite Verstärker (V2) ein zweiter Feldeffekttransistor (T2) ist, dessen Drainelektrode die zweite Ausgangselektrode (D2) ist.

3. Generator nach Anspruch 2, **dadurch gekennzeichnet, dass** die erste Steuerelektrode (G1) die Gateelektrode des ersten Feldeffekttransistors (T1) ist und dass die zweite Steuerelektrode (G2) die Gateelektrode des zweiten Feldeffektransistores (T2) ist.

4. Generator nach Anspruch 2, **dadurch gekennzeichnet, dass** der erste Verstärker (V1) ein erster Feldeffekttransistor (T4) ist, dessen Drainelektrode die erste Ausgangselektrode (D4) ist, dass der zweite Verstärker (V2) ein zweiter Feldeffekttransistor (T5) ist, dessen Drainelektrode die zweite Ausgangselektrode (D5) ist, dass die erste Steuerelektrode (S4) die Sourceelektrode des ersten Feldeffekttransistors (T4) ist und dass die zweite Steuerelektrode (S5) die Sourceelektrode des zweiten Feldeffekttransistors (T5) ist.

5. Generator nach einem der vorstehenden Ansprüche, **dadurch gekennzeichnet, dass** die beiden Verstärker (V1, V2) mit einer Gegentakt-Steuerschaltung (18) verbunden sind.

6. Gerät nach einem der vorstehenden Ansprüche, **dadurch gekennzeichnet, dass** der Induktor (19) eine Mittenanzapfung (20) aufweist, die mit einer Betriebsstromquelle (Ub) verbunden ist.

7. Gerät nach einem der vorstehenden Ansprüche 1 bis 5, **dadurch gekennzeichnet, dass** der Induktor (19) mit einem seiner beiden Enden mit einer Betriebsstromquelle (Ub) verbunden ist.

8. Generator nach einem der vorstehenden Ansprüche, **dadurch gekennzeichnet, dass** der Schalter (SW1) dazu eingerichtet ist, das Ende (23) des zweiten Kondensators (C2) wahlweise entweder hochohmig abzuschließen oder mit Masse zu verbinden.

9. Generator nach einem der vorstehenden Ansprüche, **dadurch gekennzeichnet, dass** mindestens ein weiterer Kondensatoren (C3) vorgesehen ist, der ein mit dem Induktor (19) verbundenes Ende und ein mit einem Schalter (SW2) verbundenes Ende aufweist, wobei der Schalter (SW2) dazu eingerichtet ist, das mit ihm verbundene Ende des weiteren Kondensators (C3) wahlweise entweder hochohmig abzuschließen oder mit Masse zu verbinden.

10. Generator nach einem der vorstehenden Ansprüche, **dadurch gekennzeichnet, dass** der erste Kondensator (C1) mit dem Induktor (19) parallel verbunden ist.

11. Generator nach einem der vorstehenden Ansprüche, **dadurch gekennzeichnet, dass** der erste Kondensator zwei Teilkondensatoren (C1a, C1b) umfasst, die jeweils ein mit dem Induktor (19) verbundenes Ende und ein mit Masse verbundenes Ende aufweisen.

12. Generator nach Anspruch 11, **dadurch gekennzeichnet, dass** die mit Masse verbundene Enden der Teilkondensatoren (C1a, C1b) jeweils fest mit Masse verbunden sind.

## Claims

1. Generator (12) for supply of a surgical instrument (10) with electric current,
having a first amplifier (V1) that comprises a first input electrode (G1) and a first output electrode (D1),
having a second amplifier (V2) that comprises a second input electrode (G2) and a second output electrode (D2),
having an inductor (19) that is arranged between the first and the second output electrode (D1, D2),
having a first capacitor (C1) that is connected with the inductor (19) to form an oscillating circuit,
**characterized by** a second capacitor (C2) that comprises an end (22) connected with the inductor (19) and an end (23) connected with a switch (SW1).

2. Generator according to claim 1, **characterized in that** the first amplifier (V1) is a first field effect transistor (T1), the drain electrode of which is the first output electrode (D1) and that the second amplifier (V2) is a second field effect transistor (T2), the drain electrode of which is the second output electrode (D2).

3. Generator according to claim 2, **characterized in that** the first control electrode (G1) is the gate electrode of the field effect transistor (T1) and that the second control electrode (G2) is the gate electrode of second field effect transistor (T2).

4. Generator according to claim 2, **characterized in that** the first amplifier (V1) is a first field effect transistor (T4), the drain electrode of which is the first output electrode (D4), that the second amplifier (V2) is a second field effect transistor (T5), the drain electrode of which is the second output electrode (D5), that the first control electrode (S4) is the source electrode of the first field effect transistor (T4) and that the second control electrode (S5) is the source electrode of the second field effect transistor (T5).

5. Generator according to any of the preceding claims, **characterized in that** the two amplifiers (V1, V2) are connected with a push-pull control circuit (18).

6. Apparatus according to any of the preceding claims, **characterized in that** the inductor (19) comprises a center tap (20) that is connected with an operating voltage source (Ub).

7. Apparatus according to any of the preceding claims 1 to 5, **characterized in that** the inductor (19) is connected with one of its two ends with an operating voltage source (Ub) .

8. Generator according to any of the preceding claims, **characterized in that** the switch (SW1) is configured to selectively either terminate the end (23) of a second capacitor (C2) in high ohmic manner or else to connect it to ground.

9. Generator according to any of the preceding claims, **characterized in that** at least one additional capacitor (C3) is provided that comprises an end connected with the inductor (19) and an end connected with a switch (SW2), wherein the switch (SW2) is configured to selectively either terminate the end of the additional capacitor (C3) connected to it in high ohmic manner or else connect it to ground.

10. Generator according to any of the preceding claims, **characterized in that** the first capacitor (C1) is connected in parallel with the inductor (19).

11. Generator according to any of the preceding claims, **characterized in that** the first capacitor comprises two sub-capacitors (C1a, C1b) that respectively comprise one end connected to the inductor (19) and one end connected to ground.

12. Generator according to claim 11, **characterized in that** the ends of the sub-capacitors (C1a, C1b) connected to ground are respectively inseparably connected to ground.

## Revendications

1. Générateur (12) destiné à l'alimentation en courant électrique d'un instrument chirurgical (10),
comprenant un premier amplificateur (V1) qui présente une première électrode d'entrée (G1) et une première électrode de sortie (D1),
comprenant un deuxième amplificateur (V2), qui présente une deuxième électrode d'entrée (G2) et une deuxième électrode de sortie (D2),
comprenant un inducteur (19) qui est placé entre la première et la deuxième électrode de sortie (D1, D2),
comprenant un premier condensateur (C1) qui est relié à l'inducteur (19) pour former un circuit oscillant,
**caractérisé par**
un deuxième condensateur (C2) qui présente une extrémité (22) reliée à l'inducteur (19) et une extrémité (23) reliée à un interrupteur (SW1).

2. Générateur selon la revendication 1, **caractérisé en ce que** le premier amplificateur (V1) est un premier transistor à effet de champ (T1) dont l'électrode drain est la première électrode de sortie (D1), et **en ce que** le deuxième amplificateur (V2) est un deuxième transistor à effet de champ (T2) dont l'électrode drain est la deuxième électrode de sortie (D2).

3. Générateur selon la revendication 2, **caractérisé en ce que** la première électrode de commande (G1) est l'électrode grille du premier transistor à effet de champ (T1), et **en ce que** la deuxième électrode de commande (G2) est l'électrode grille du deuxième transistor à effet de champ (T2).

4. Générateur selon la revendication 2, **caractérisé en ce que** le premier amplificateur (V1) est un premier transistor à effet de champ (T4) dont l'électrode drain est la première électrode de sortie (D4), **en ce que** le deuxième amplificateur (V2) est un deuxième transistor à effet de champ (T5) dont l'électrode drain est la deuxième électrode de sortie (D5), **en ce que** la première électrode de commande (S4) est l'électrode source du premier transistor à effet de champ (T4), et **en ce que** la deuxième électrode de commande (S5) est l'électrode source du deuxième transistor à effet de champ (T5).

5. Générateur selon l'une des revendications précédentes, **caractérisé en ce que** les deux amplificateurs (V1, V2) sont reliés à un circuit de commande symétrique (18).

6. Appareil selon l'une des revendications précédentes, **caractérisé en ce que** l'inducteur (19) présente une prise médiane (20) qui est reliée à une source de courant de service (Ub).

7. Appareil selon l'une des revendications précédentes 1 à 5, **caractérisé en ce que** l'inducteur (19) est relié par une de ses deux extrémités à une source de courant de service (Ub).

8. Générateur selon l'une des revendications précédentes, **caractérisé en ce que** l'interrupteur (SW1) est conçu pour, au choix, terminer avec une haute impédance l'extrémité (23) du deuxième condensateur (C2) ou la relier à la masse.

9. Générateur selon l'une des revendications précédentes, **caractérisé en ce qu'**il est prévu au moins un condensateur (C3) supplémentaire qui présente une extrémité reliée à l'inducteur (19) et une extrémité reliée à un interrupteur (SW2), l'interrupteur (SW2) étant conçu pour, au choix, terminer avec une haute impédance l'extrémité du condensateur (C3) supplémentaire à laquelle il est raccordé, ou la relier à la masse.

10. Générateur selon l'une des revendications précédentes, **caractérisé en ce que** le premier condensateur (C1) est relié en parallèle à l'inducteur (19).

11. Générateur selon l'une des revendications précédentes, **caractérisé en ce que** le premier condensateur comprend deux condensateurs élémentaires (C1a, C1b) qui présentent chacun une extrémité reliée à l'inducteur (19) et une extrémité reliée à la masse.

12. Générateur selon la revendication 11, **caractérisé en ce que** les extrémités des condensateurs élémentaires (C1a, C1b) qui sont reliées à la masse sont chacune reliées solidement à la masse.
